(19) **Europäisches Patentamt**
**European Patent Office**
**Office européen des brevets**

(11) **EP 4 694 775 B1**

(12) **EUROPEAN PATENT SPECIFICATION**

(45) Date of publication and mention
of the grant of the patent:
**05.08.2026 Bulletin 2026/32**

(21) Application number: **24718359.3**

(22) Date of filing: **02.04.2024**

(51) International Patent Classification (IPC):
***A61B 6/03*** (2006.01) ***A61B 6/00*** (2024.01)

(52) Cooperative Patent Classification (CPC):
**A61B 6/482; A61B 6/032; A61B 6/5217;**
A61B 6/405; A61B 6/4241

(86) International application number:
**PCT/EP2024/058853**

(87) International publication number:
**WO 2024/213426 (17.10.2024 Gazette 2024/42)**

(54) **PREDICTING EFFECTIVE ENERGY VALUES**

VORHERSAGE EFFEKTIVER ENERGIEWERTE

PRÉDICTION DE VALEURS D'ÉNERGIE EFFICACE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **10.04.2023 RU 2023108950**

(43) Date of publication of application:
**18.02.2026 Bulletin 2026/08**

(73) Proprietor: **Koninklijke Philips N.V.**
**5656 AE Eindhoven (NL)**

(72) Inventors:
• **DAERR, Heiner**
**5656 AG Eindhoven (NL)**
• **SCHNELLBÄCHER, Nikolas David**
**5656 AG Eindhoven (NL)**
• **POUDEL, Prabal**
**5656 AG Eindhoven (NL)**
• **TSANDA, Artyom**
**5656 AG Eindhoven (NL)**

(74) Representative: **Philips Intellectual Property &**
**Standards**
**High Tech Campus 34**
**5656 AE Eindhoven (NL)**

(56) References cited:
• **TATSUGAMI FUMINARI ET AL: "Dual-energy CT:
minimal essentials for radiologists", JAPANESE
JOURNAL OF RADIOLOGY, SPRINGER JAPAN,
TOKYO, vol. 40, no. 6, 4 January 2022
(2022-01-04), pages 547 - 559, XP037870835,
ISSN: 1867-1071, [retrieved on 20220104], DOI:
10.1007/S11604-021-01233-2**
• **SO AARON ET AL: "Spectral Computed
Tomography: Fundamental Principles and
Recent Developments", KOREAN JOURNAL OF
RADIOLOGY : OFFICIAL JOURNAL OF THE
KOREAN
RADIOLOGICAL SOCIETY, vol. 22, no. 1, 1
January 2021 (2021-01-01), pages 86,
XP093175665, ISSN: 1229-6929, Retrieved from
the Internet <URL:https://kjronline.org/DOIx.
php?id=10.3348/kjr.2020.0144> DOI: 10.3348/
kjr.2020.0144**
• **MILLNER MICHAEL R. ET AL: "Determination of
effective energies in CT calibration", MEDICAL
PHYSICS, 31 December 1978 (1978-12-31), pages
543 - 545, XP093175837**

EP 4 694 775 B1

**(Cont. next page)**

- **LI YINSHENG ET AL: "A quality-checked and physics-constrained deep learning method to estimate material basis images from single-kV contrast-enhanced chest CT scans", MEDICAL PHYSICS., vol. 50, no. 6, 23 March 2023 (2023-03-23), US, pages 3368 - 3388, XP093175906, ISSN: 0094-2405, Retrieved from the Internet <URL:https://onlinelibrary.wiley.com/doi/full-xml/10.1002/mp.16352> DOI: 10.1002/mp.16352**
- **ZIMMERMAN KEVIN C ET AL: "Experimental investigation of neural network estimator and transfer learning techniques for K-edge spectral CT imaging", MEDICAL PHYSICS., vol. 47, no. 2, 6 January 2020 (2020-01-06), US, pages 541 - 551, XP093175903, ISSN: 0094-2405, Retrieved from the Internet <URL:https://onlinelibrary.wiley.com/doi/full-xml/10.1002/mp.13946> DOI: 10.1002/mp.13946**
- **SCHMIDT TALY GILAT ET AL: "A Spectral CT Method to Directly Estimate Basis Material Maps From Experimental Photon-Counting Data", IEEE TRANSACTIONS ON MEDICAL IMAGING, vol. 36, no. 9, 1 September 2017 (2017-09-01), USA, pages 1808 - 1819, XP093175848, ISSN: 0278-0062, DOI: 10.1109/TMI.2017.2696338**

**Description**

FIELD OF THE INVENTION

This invention relates to the field of predicting an effective energy value of a conventional CT image, and in particular to the field of training a neural network model for predicting an effective energy value of a conventional CT image.

BACKGROUND OF THE INVENTION

Dual energy CT imaging is well established. The simplest approach is to acquire two separate scans with two different kVp settings, i.e. taken at two different photon energies. Material decomposition can then be accomplished in the projection domain or the image domain. Typically, there is a small but unneglectable motion between the two scans due to the time delay between them. A registration of the low and high data sets is required, and can best be performed in the image domain. After image registration, an image-based material decomposition of the CT images can be applied using the now spatially matched images. Image-based material decomposition of CT images is also well established in the art.

In the theory of image-domain material decomposition, the linear attenuation coefficients of each pixel in the low and high CT images $\mu_{H,L}(x, y, z) = \mu_{H,L}$ are approximated by a linear combination of the pixel values in the images of the basis materials; here photoelectric effect (*ph*) and Compton Effect or scatter (*sc*):

$$\begin{pmatrix} \mu_H \\ \mu_L \end{pmatrix} = \begin{pmatrix} \mu^{ph}(E_H) & \mu^{sc}(E_H) \\ \mu^{ph}(E_L) & \mu^{sc}(E_L) \end{pmatrix} \begin{pmatrix} ph \\ sc \end{pmatrix} = A \begin{pmatrix} ph \\ sc \end{pmatrix} \qquad (1)$$

$E_H$ and $E_L$ denote the effective energy of the low and the high pixel values. In the case of mono-energetic images, the matrix A is true for all image pixels. The effective energy depends on the attenuation characteristics of the object and thus changes for each CT image slice. The images of the basis materials are calculated from the low and high images pixelwise using the inverse matrix $A^{-1}$ of A:

$$\begin{pmatrix} ph \\ sc \end{pmatrix} = A^{-1} \begin{pmatrix} \mu_H \\ \mu_L \end{pmatrix} \qquad (2)$$

The matrix $A^{-1}$ depends on the scanner (spectrum, detector and processing) and the composition, size and shape of the object due to beam hardening. Assuming the low and high images are mono-energetic and the effective/mono energies $E_H$ and $E_L$ are known the matrix A is given by linear attenuation coefficients of the basis materials:

$$A(ph,\ sc,\ E_H,\ E_L) = \begin{pmatrix} \mu^{ph}(E_H) & \mu^{sc}(E_H) \\ \mu^{ph}(E_L) & \mu^{sc}(E_L) \end{pmatrix} \qquad (3)$$

One main challenge for image-based material decomposition, however, is finding the effective energies of the CT images in order to calculate the right transfer function from the conventional input CT images to the material images. In clinical practice, tedious system calibration procedures, from which the effective energy can be found, should be avoided as they slow down the workflow and are subject to drifts.

Hence, there is a need for a solution for predicting an effective energy value of a conventional CT image.

The article « Determination of effective energies in CT calibration" by Millner et al., Med Phys. 1978 Nov-Dec;5(6):543-5. doi: 10.1118/1.594488, describes methods for determining the effective X-ray energies used in the calibration of computed tomography (CT) systems.

The article "A quality-checked and physics-constrained deep learning method to estimate material basis images from single-kV contrast-enhanced chest CT scans", by Li Yinsheng et al, Medical Physics., vol. 50, no. 6, 23 March 2023, pages 3368-3388, ISSN: 0094-2405, DOI: 10.1002/mp.16352, describes a method to for predicting an effective energy value of a conventional CT image.

The article "Experimental investigation of neural network estimator and transfer learning techniques for K-edge spectral CT imaging", Medical Physics, vol. 47, no. 2, 6 January 2020, pages 541-551, ISSN: 0094-2405, DOI: 10.1002/mp.13946, describes a machine learning algorithm for spectral CT imaging of K-edge contrast agents using simulated and experimental measurements.

SUMMARY OF THE INVENTION

The invention is defined by the claims.

According to examples in accordance with an aspect of the invention, there is provided a method for training a neural network model for predicting an effective energy value of a conventional CT image.

The method comprises: training a neural network model using a training algorithm configured to receive an array of training inputs and respective known outputs, wherein a training input comprises a spectral scanner mono-energetic CT image and a respective known output comprises the effective energy value of the spectral scanner mono-energetic CT image.

Proposed concepts thus aim to provide schemes, solutions, concept, designs, methods and systems pertaining to training a neural network model for predicting an effective energy value of a conventional CT image.

In particular, embodiments aim to utilize mono-energetic CT images from spectral scanners, of which the effective energy values are known, in order to train a neural network model which can then be used to predict an effective energy value of a conventional CT image, i.e. typically not mono-energetic. In other words, training of the neural network model is done using mono-energetic images from a spectral scanner, however, the trained neural network model may then be used to predict the effective energy value of a conventional CT image obtained from either a spectral or a conventional scanner. By training a neural network model to predict the effective energy value of a CT image using the image alone, a method of estimating the effective energy of a CT image may be provided that requires no system calibration and no additional system or image information. Not even the actual kVp setting information of the scan is needed. Calibration procedures during clinical practice may then be avoided.

In other words, it is proposed that a neural network model may be trained using a training algorithm, wherein the training algorithm is configured to receive spectral scanner mono-energetic CT images as the training inputs, and the effective energy values of the mono-energetic CT images as the respective known outputs. This may ultimately provide a neural network model capable of estimating the effective energy values of conventional (i.e. non-mono-energetic) CT images from the images alone, thus allowing a transfer function to be calculated for the images and enabling material decomposition. This method also avoids CT system calibration procedures which slow down workflow and are often subject to drifts. Implementing this purely data driven solution circumvents calibrations and improves the ease of obtaining an estimated effective energy value of a conventional CT image.

By training a neural network model to predict an effective energy value from a single input CT image, ultimately a method of estimating an effective energy value of a conventional CT image from purely the image alone may be provided.

The use of spectral scanner mono-energetic CT images, of which the effective energy values are known, to train a neural network model allows the neural network model to become proficient at estimating effective energy values even of non-mono-energetic CT images, i.e. conventional CT images. In other words, it has been realized that mono-energetic CT images, which may be obtained with a spectral scanner, may be leveraged in order to train a neural network model so that it may predict effective energy values even of conventional CT images.

Ultimately, a way to train a neural network model so that it may predict an effective energy value of a conventional CT image from the image alone is provided, so that the effective energy value of a CT image may be estimated without any additional or contextual information. The effective energy value of a CT image may then be leveraged for future purposes, for instance to calculate a transfer function for material decomposition.

In some embodiments, the array of training inputs comprise spectral scanner mono-energetic CT images from 40keV to 140keV with a step-size of 0.1keV or more. This is a typical range of the effective energy of conventional CT images and enables the model to estimate the effective energy of a CT image to an accuracy of 0.1keV.

In some embodiments, the spectral scanner mono-energetic CT images are specifically whole single axial CT images.

In some embodiments, the array of training inputs further comprise adjusted spectral scanner mono-energetic CT images, wherein the adjustment comprises slice-flipping, rotating, shifting, zooming, cropping, and using different noise levels. This increases the amount of training data the neural network is trained on, and also allows the training to be made invariant against varying image characteristics due to reconstruction parameters or anatomical changes.

In some embodiments, the neural network model comprises a convolutional neural network (CNN) or a machine learning regression model or a classification model. CNNs have been proven to be particularly successful at analyzing images, and have been found to be able to classify images with a much lower error rate than other types of neural network. Regression models have been found to be particularly useful for analyzing the relation between independent features, e.g. the pixels of a CT image, and a dependent variable, e.g. the effective energy. Classification models are particularly useful for assigning data to discrete classes based on a specific set of features.

In some embodiments, the array of training inputs further comprises corresponding aggregated feature vectors of each respective spectral scanner mono-energetic CT image. Using corresponding aggregated feature vectors alongside the mono-energetic CT images has been found to improve performance of the neural network model.

In some embodiments, the aggregated feature vectors are pre-processed using a principle components analysis (PCA). By focusing on the most significant feature vector components, it has been found that the training performance of the neural network model can be further improved.

In some embodiments, the aggregated feature vectors comprise histograms of the Hounsfield units (HU) values within each respective spectral scanner mono-energetic CT image. It has been found that the particular use of histograms of the HU values within the CT images during training can significantly improve performance of the neural network model.

There is also provided a method for estimating an effective energy value of a conventional CT image, the method comprising: obtaining a first CT image; and processing the first CT image, using a neural network model trained in accordance with any herein disclosed method, to generate a first estimated effective energy value of the first CT image.

After training in accordance with any method described above, the neural network model may then be able to actually process a CT image and generate an estimated effective energy value of the processed CT image.

In some embodiments, the method for estimating an effective energy value of a conventional CT image further comprises using the first estimated effective energy value in a further image-processing step of the first CT image such as a bone beam hardening correction. Once an estimated effective energy value of a CT image has been generated, the value may then be used to improve image-processing steps. For example, the estimated effective energy value of a CT image can be used in a bone beam hardening correction of the CT image.

In some embodiments, the method for estimating an effective energy value of a conventional CT image further comprises segmenting the first CT image into multiple CT sub-images, and processing the multiple CT sub-images using the neural network model to generate estimated effective energy values for each CT sub-image. This may allow for the fact that the effective energy value might vary within a conventional CT image to be taken into account. For example, CT images with strong beam hardening artefacts are more likely to result in images where the effective energy value varies within the image. The estimated effective energy values of each sub-image may then be utilized to calculate different local transfer functions for each sub-image, which may then be leveraged to apply material decomposition to each sub-image using each local transfer function respectively.

In some embodiments, the method for estimating an effective energy value of a conventional CT image further comprises obtaining a second CT image, processing the second CT image, using the neural network model, to generate a second estimated effective energy value of the second CT image, and calculating a transfer function based on the first estimated effective energy value of the first CT image and the second estimated effective energy value of the second CT image. This may be of particular use in the case of dual energy CT imaging, wherein two CT images are generated in each scan, one of lower energy and one of higher. Once effective energy values have been estimated for both CT images, a transfer function can then be calculated based on the estimated effective energy values.

In some embodiments, the above method further comprises performing a material decomposition based on the first CT image and the second CT image using the calculated transfer function. Spectral imaging enabled by material decomposition is an important application of CT images.

In some embodiments, the method for estimating an effective energy value of a conventional CT image comprising obtaining a second CT image further comprises obtaining at least a third CT image, processing the at least third CT image, using the neural network model, to generate a third estimated effective energy value of the at least third CT image, calculating the transfer function further based on the third estimated effective energy value of the at least third CT image, and performing a material decomposition based on the first CT image, the second CT image and the third CT image using the calculated transfer function. This may be of particular use in the case of multi-energy material decomposition, wherein more than two images are generated for each scan, for instance in photon counting CT systems.

There is also provided a computer program comprising code means for implementing any herein disclosed method when said program is run on a processing system.

There is also provided a system for training a neural network model for predicting an effective energy value of a conventional CT image, the system comprising: a processor arrangement configured to: train a neural network model using a training algorithm configured to receive an array of training inputs and respective known outputs, wherein a training input comprises a spectral scanner mono-energetic CT image and a respective known output comprises the effective energy value of the spectral scanner mono-energetic CT image.

There is also provided a system for estimating an effective energy value of a conventional CT image, the system comprising a processor arrangement configured to a first CT image, process (330) the first CT image, and use the neural network model trained in accordance with any of the methods of any of claims 1 to 7, to generate a first estimated effective energy value of the first CT image.

Thus, there may be proposed concepts for training a neural network model for predicting an effective energy value of a conventional CT image.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 is a schematic illustration of an imaging system such as a CT scanner;
Fig. 2A is a simplified flow diagram of a method for training a neural network model for predicting an effective energy value of a conventional CT image;
Fig. 2B is a more in-depth explanation of the method of Fig. 2A;
Fig. 3 is a flow diagram of a method for estimating an effective energy value of a conventional CT image;
Fig. 4 is a flow diagram of a method for performing material decomposition based on multiple CT images;
Fig. 5 is a simplified block diagram of a system for training a neural network model for predicting an effective energy value of a conventional CT image; and
Fig. 6 illustrates an example of a computer within which one or more parts of an embodiment may be employed.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figures are merely schematic and are not drawn to scale. It should also be understood that the same reference numerals are used throughout the Figures to indicate the same or similar parts.

Implementations in accordance with the present disclosure relate to various techniques, methods, schemes and/or solutions pertaining to training a neural network model for predicting an effective energy value of a conventional CT image. According to proposed concepts, a number of possible solutions may be implemented separately or jointly. That is, although these possible solutions may be described below separately, two or more of these possible solutions may be implemented in one combination or another.

Embodiments of the invention aim to utilize mono-energetic CT images from spectral scanners, of which the effective energy values are known, in order to train a neural network model which can then be used to predict an effective energy value of a conventional (i.e. non-mono-energetic) CT image. By training a neural network model to predict the effective energy value of a CT image using the image alone, a method of estimating the effective energy of a CT image may be provided that requires no system calibration and no additional system or image information. Not even the actual kVp setting information of the scan is needed. Calibration procedures during clinical practice may then be avoided.

Proposed concepts thus aim to provide schemes, solutions, concept, designs, methods and systems pertaining to training a neural network model for predicting an effective energy value of a conventional CT image.

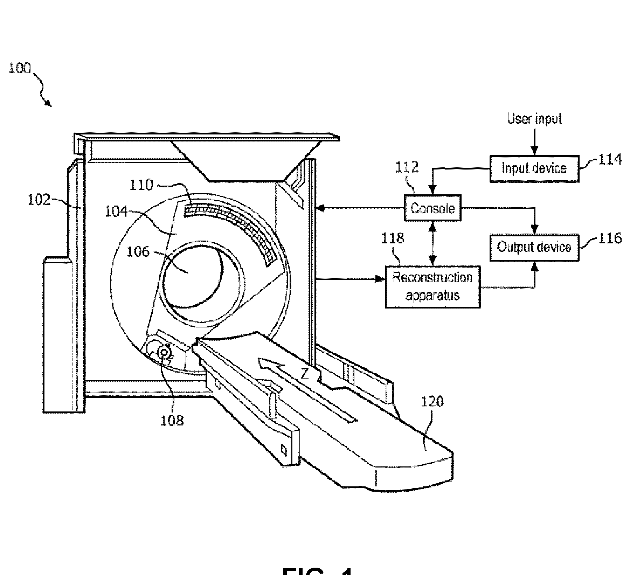

Fig. 1 schematically illustrates an imaging system 100 such as a CT scanner configured for spectral (multi-energy) imaging, such as would be used to acquire the training data. The imaging system 100 includes a generally stationary gantry 102 and a rotatable gantry 104, which is rotatably supported by the stationary gantry and rotates around an examination region 106 about a z-axis. A subject support 120, such as a couch, supports an object or subject in the examination region.

A radiation source 108, such as an x-ray tube, is rotatably supported by the rotatable gantry 104, rotates with the rotatable gantry, and emits radiation that traverses the examination region 106. In one instance, the radiation source includes a single broad spectrum x-ray tube. In another instance, the radiation source includes a single x-ray tube configured to switch between at least two different emission voltages (e.g., 80 kVp and 140 kVp) during scanning. In yet another instance, the radiation source includes two or more x-ray tubes configured to emit radiation having different mean spectra. In still another instance, the radiation source includes a combination thereof.

A radiation sensitive detector array 110 subtends an angular arc opposite the radiation source 108 across the examination region 106. The radiation sensitive detector array detects radiation traversing the examination region and generates an electrical signal(s) (projection data) indicative thereof. Where the radiation source includes a single broad spectrum x-ray tube, the radiation sensitive detector array includes energy-resolving detectors (e.g., direct conversion photon counting detectors, at least two sets of scintillators with different spectral sensitivities (multi-layer), etc.). With kVp switching and multi-tube configurations, the detector array can include single layer detectors, direct conversion photon counting detectors, and/or multi-layer detectors. The direct conversion photon counting detectors may include a conversion material such as CdTe, CdZnTe, Si, Ge, GaAs, or other direct conversion material. An example of multi-layer detector includes a double decker detector such as the double decker detector described in US 7,968,853 B2.

A reconstruction apparatus 118 receives spectral projection data from the detector array 110 and reconstructs spectral volumetric image data such as sCCTA image data, a high-energy image, a low energy image, a photoelectric image, a Compton scatter image, an iodine image, a calcium image, a virtual non-contrast image, a bone image, a soft tissue image, and/or other basis material image. The reconstruction apparatus can also reconstruct non-spectral volumetric image data, e.g., by combining spectral projection data and/or spectral volumetric image data. Generally, the spectral projection data and/or spectral volumetric image data will include data for at least two different energies and/or energy ranges.

A console 112 serves as an operator console. The console is operably connected to an output device 116 such as a monitor and an input device 114 such as a keyboard, mouse, etc. Software resident on the console 112 allows the operator to interact with and/or operate the imaging system 100 via a graphical user interface (GUI) or otherwise.

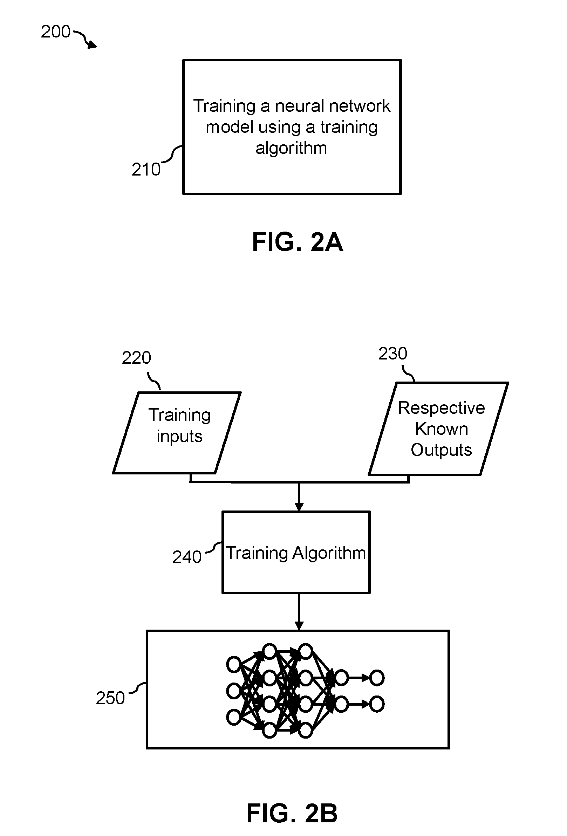

Referring now to Figs. 2A and 2B, there is depicted a flow diagram of a method 200 for training a neural network model 250 for predicting an effective energy value of a conventional CT image, according to a proposed embodiment.

The method comprises step 210 of training a neural network model 250 using a training algorithm 240 configured to receive an array of training inputs 220 and respective known outputs 230, wherein a training input comprises a spectral scanner mono-energetic CT image and a respective known output comprises the effective energy value of the spectral scanner mono-energetic CT image. The effective energy of a CT image depends on the attenuation characteristics of the object being scanned and thus changes for each CT image, even for the same patient, for instance an axial scan of a patient's abdomen will have a different effective energy to an axial scan of their legs. The trained neural network model will be capable of processing and estimating effective energy values of CT images even from different CT systems to the CT systems its training data were obtained with.

In some embodiments, the neural network model 250 comprises a convolutional neural network (CNN) or a machine learning regression model or a classification model. CNNs have been proven to be particularly successful at analyzing images, and have been found to be able to classify images with a much lower error rate than other types of neural network. Regression models have been found to be particularly useful for analyzing the relation between independent features, e.g. the pixels of a CT image, and a dependent variable, e.g. the effective energy. Classification models are particularly useful for assigning data to discrete classes, e.g. the effective energy value, based on a specific set of features. Example CNN models that may be of use are DenseNet, ResNet-like, or EfficientNet. A machine learning regression model that may be of use is Random Forest.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

There are several types of neural network, such as convolutional neural networks (CNNs) and recurrent neural networks (RNNs). This exemplary embodiment of the present invention employs CNN-based learning algorithms, because CNNs have proved to be particularly successful at analyzing videos, and are able to classify frames within videos with a much lower error rate than other types of neural network.

CNNs typically contain several layers, including a convolutional layer, a pooling layer, and a fully connected layer. The convolutional layer consists of a set of learnable filters and extracts features from the input. The pooling layer is a form of non-linear down-sampling, reducing the data size by combining the outputs of a plurality of neurons in one layer into a single neuron in the next layer. The fully connected layer connects each neuron in one layer to all the neurons in the next layer.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. $\pm1\%$) to the training output data entries. This is commonly known as a supervised learning technique.

For example, weightings of the mathematical operation of each neuron may be modified until the error converges. Known methods of modifying a neural network include gradient descent, backpropagation algorithms and so on.

The training input data entries 220 for the CNN which may be used in method 200 correspond to spectral scanner mono-energetic CT images. The training output data entries 230 correspond to the effective energy values of each respective spectal scanner mono-energetic CT image. Further, several pre-processing methods may be employed to improve the training samples. In other words, the first CNN can be trained using a training algorithm 240 configured to receive an array of training inputs and respective known outputs, wherein a training input comprises a spectral scanner mono-energetic CT image and a respective known output comprises the effective energy value of the spectral scanner mono-energetic CT image. In this way, the CNN is trained to output the effective energy value of a spectral scanner mono-energetic CT image when provided with the spectral scanner mono-energetic CT image.

The spectral scanner mono-energetic CT images can be readily created using spectral CT systems by creating the corresponding mono-energetic images for various mono energy levels. It is noted that real human patient data is required, and not merely phantom data.

In some embodiments, the array of training inputs 220 comprise spectral scanner mono-energetic CT images from 40keV to 140keV with a step-size of 0.1keV or more. This is a typical range of the effective energy of conventional CT images and enables the model to estimate the effective energy of a CT image to an accuracy of 0.1keV.

In some embodiments, the spectral scanner mono-energetic CT images are specifically whole single axial CT images.

In some embodiments, the array of training inputs 220 further comprise adjusted spectral scanner mono-energetic CT images, wherein the adjustment comprises slice-flipping, rotating, shifting, zooming, cropping, and using different noise levels. For instance, a low-pass filter may be applied to the mono-energetic CT images. This increases the amount of training data the neural network model 250 is trained on, and also allows the training to be made invariant against varying image characteristics due to reconstruction parameters or anatomical changes. In other words, providing multiple versions of a CT image at a given target energy makes the training agnostic to an extent. This technique is also called data augmentation.

In some embodiments, the array of training inputs 220 further comprises corresponding aggregated feature vectors of each respective spectral scanner mono-energetic CT image. Using corresponding aggregated feature vectors alongside the mono-energetic CT images has been found to improve performance of the neural network model 250. For example, the corresponding aggregated feature vectors of each respective spectral scanner mono-energetic CT image may be utilized in multi-layer perceptron (MLP), random decision forests or adaptive boosting during training. In some embodiments, the aggregated feature vectors may suffice by themselves, essentially replacing the original CT images.

In some embodiments, the aggregated feature vectors are pre-processed using a principle components analysis (PCA). By focusing on the most significant feature vector components, it has been found that the training performance of the neural network model 250 can be further improved.

In some embodiments, the aggregated feature vectors comprise histograms of the Hounsfield units (HU) values within each respective spectral scanner mono-energetic CT image. It has been found that the particular use of histograms of the HU values within the CT images during training can significantly improve performance of the neural network model 250.

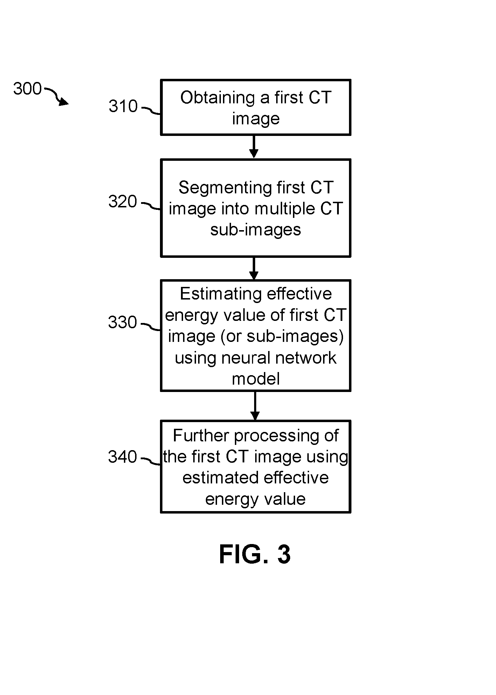

Referring now to Fig. 3, there is provided a diagram of a method 300 for estimating an effective energy value of a conventional CT image.

In step 310, a first CT image is obtained. The first CT image does not have to be mono-energetic, and may be any conventional CT image. The conventional CT image may include scatter corrections and beam hardening corrections.

In step 320, the first CT image is segmented into multiple CT sub-images. This allows for the fact that the effective energy might vary within the first CT image. For example, CT images with strong beam hardening artefacts are more likely to result in images where the effective energy varies within the image. However, step 320 is optional and the method may continue with a single whole first CT image, i.e. without step 320 taking place. In step 330, the multiple CT sub-images (or single whole first CT image) are processed using the neural network trained in method 200 to generate estimated effective energy values for each CT sub-image (or to generate one estimated effective energy value for the entire first CT image). In some cases, the sub-images may each have the same estimated effective energy value, in which case, one effective energy value may be taken as the value for the entire image.

In step 340, the estimated effective energy value of the first CT image (or sub-images) may be used in a further image-processing step of the first CT image. For example, the estimated effective energy value of a CT image may be used in a bone beam hardening correction of the CT image.

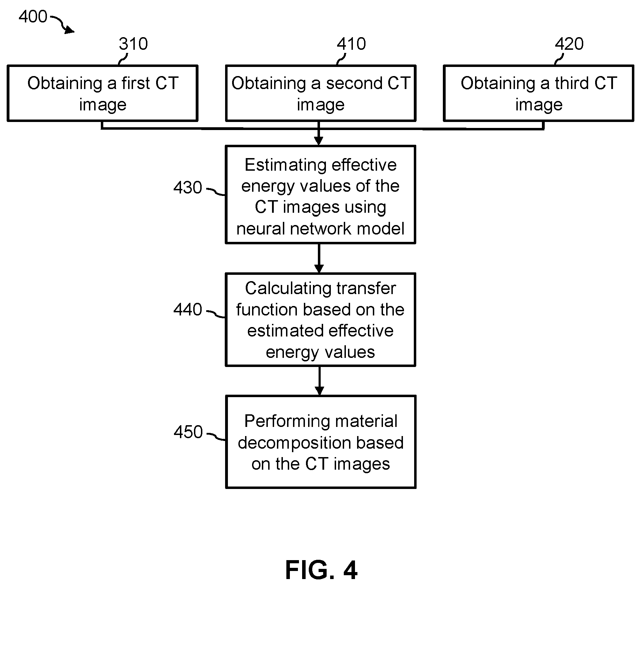

Referring now to Fig. 4, there is provided a diagram of a method 400 for performing material decomposition based on multiple (i.e. at least two) CT images.

In step 310, a first CT image is obtained. In step 410, a second CT image is obtained. In step 420, at least a third CT image is obtained.

In step 430, the first, second, and at least a third CT images are processed, using the neural network trained in method 200 to generate a first, second and third effective energy value of the first, second, and at least a third CT images respectively.

In step 440, a transfer function is calculated based on the first, second, and third estimated effective energy values of the first, second, and at least a third CT images respectively. Estimating the effective energy values of two CT images and calculating a transfer function based on both estimated effective energy values may be of particular use in the case of dual energy CT imaging, wherein two conventional CT images are generated in each scan, one of lower energy and one of higher. Examples of dual energy CT systems are fast kVp-switching systems, dual layer detector systems, and dual source systems. The addition of at least a third CT image, and the subsequent estimation of its effective energy value and use of that value in the calculation of a transfer function may be of particular use in the case of multi-energy material decomposition, wherein more than two images are generated for each scan, for instance in photon counting CT systems.

In step 450, a material decomposition based on the first CT image, the second CT image and at least a third CT image is performed using the transfer function calculated in step 440. In cases in which only two CT images are obtained, the material decomposition is performed based on only the first and second CT images using the transfer function calculated using the first and second estimated effective energy values of the first and second CT images respectively. Spectral imaging enabled by material decomposition is an important application of CT images.

In more detail, for each energy channel $e$, a conventional CT image must be reconstructed and the effective energies $E_e$ for each energy channel, i.e. for each CT image, needs to be estimated, for example, using any of the herein disclosed methods. In the case where the number of materials in the CT images (e.g. 2) is less than the number of energy channels (e.g. 3), a forward model can be given by:

$$\begin{pmatrix} \mu_1 \\ \mu_2 \\ \mu_3 \end{pmatrix} = \begin{pmatrix} \mu^{ph}(E_1) & \mu^{sc}(E_1) \\ \mu^{ph}(E_2) & \mu^{sc}(E_2) \\ \mu^{ph}(E_3) & \mu^{sc}(E_3) \end{pmatrix} \begin{pmatrix} ph \\ sc \end{pmatrix} = A \begin{pmatrix} ph \\ sc \end{pmatrix}$$

(4)

Equation (4) may then be inverted by use of the pseudo inverse $A^T A$ or the weighted pseudo inverse $A^T C^{-1} A$ with $C$ being the covariance matrix between the input CT images. The weighted pseudo inverse is an embodiment of the transfer function mentioned above.

Taking the example of obtaining and processing two CT images, effective energy values may be estimated for each image pair at a given z-position. The matrix given in equation 3 may then be formulated, inverted, and applied to the image pair to calculate the materials images $ph$ and $sc$ for the given z-position.

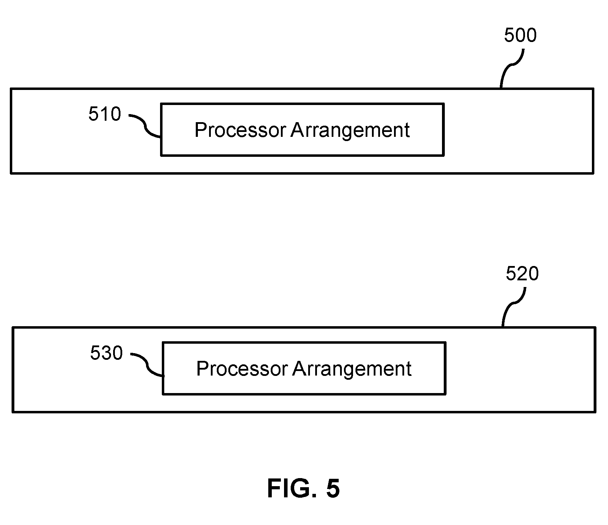

Referring now to Fig. 5, there is depicted a simplified block diagram of a system 500 for training a neural network model for predicting an effective energy value of a conventional CT image, according to an embodiment. The system configured for training a neural network model for predicting an effective energy value of a conventional CT image comprises a processor arrangement 510.

The processor arrangement 510 is configured to train a neural network model using a training algorithm configured to receive an array of training inputs and respective known outputs, wherein a training input comprises a spectral scanner mono-energetic CT image and a respective known output comprises the effective energy value of the spectral scanner mono-energetic CT image.

Referring further to Fig. 5, there is also depicted a simplified block diagram of a system 520 for estimating, using a neural network model, an effective energy value of a conventional CT image. The system uses a neural network model trained to predict an effective energy value of a conventional CT image according to the invention. The system 520 comprises a processor arrangement 530.

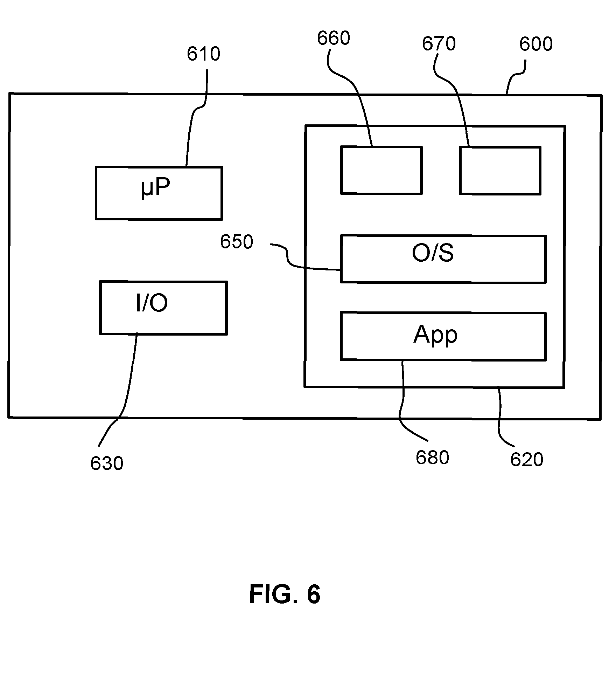

Referring now to Fig. 6, illustrates an example of a computer 600 within which one or more parts of an embodiment may be employed. Various operations discussed above may utilize the capabilities of the computer 600. In this regard, it is to be understood that system functional blocks can run on a single computer or may be distributed over several computers and locations (e.g. connected via internet).

The computer 600 includes, but is not limited to, PCs, workstations, laptops, PDAs, palm devices, servers, storages, and the like. Generally, in terms of hardware architecture, the computer 600 may include one or more processors 610, memory 620 and one or more I/O devices 630 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 610 is a hardware device for executing software that can be stored in the memory 620. The processor 610 can be virtually any custom made or commercially available processor, a central processing unit (CPU), a digital signal processor (DSP), or an auxiliary processor among several processors associated with the computer 600, and the processor 610 may be a semiconductor based microprocessor (in the form of a microchip) or a microprocessor.

The memory 620 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 620 may incorporate electronic, magnetic, optical, and/or other types of storage media. Note that the memory 620 can have a distributed architecture, where various components are situated remote from one another, but can be accessed by the processor 610.

The software in the memory 620 may include one or more separate programs, each of which comprises an ordered listing of executable instructions for implementing logical functions. The software in the memory 620 includes a suitable operating system (O/S) 650, compiler 660, source code 670, and one or more applications 680 in accordance with exemplary embodiments. As illustrated, the application 680 comprises numerous functional components for implementing the features and operations of the exemplary embodiments. The application 680 of the computer 600 may represent various applications, computational units, logic, functional units, processes, operations, virtual entities, and/or modules in accordance with exemplary embodiments, but the application 680 is not meant to be a limitation.

The operating system 650 controls the execution of other computer programs, and provides scheduling, input-output control, file and data management, memory management, and communication control and related services. It is contemplated by the inventors that the application 680 for implementing exemplary embodiments may be applicable on all commercially available operating systems.

Application 680 may be a source program, executable program (object code), script, or any other entity comprising a set of instructions to be performed. When a source program, then the program is usually translated via a compiler (such as the compiler 660), assembler, interpreter, or the like, which may or may not be included within the memory 620, so as to operate properly in connection with the O/S 650. Furthermore, the application 680 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, NET, and the like.

The I/O devices 630 may include input devices such as, for example but not limited to, a mouse, keyboard, scanner, microphone, camera, etc. Furthermore, the I/O devices 630 may also include output devices, for example but not limited to a printer, display, etc. Finally, the I/O devices 630 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 600 is a PC, workstation, intelligent device or the like, the software in the memory 620 may further include a basic input output system (BIOS) (omitted for simplicity). The BIOS is a set of essential software routines that initialize and test hardware at start-up, start the O/S 650, and support the transfer of data among the hardware devices. The BIOS is stored in some type of read-only-memory, such as ROM, PROM, EPROM, EEPROM or the like, so that the BIOS can be executed when the computer 800 is activated.

When the computer 600 is in operation, the processor 610 is configured to execute software stored within the memory 620, to communicate data to and from the memory 620, and to generally control operations of the computer 600 pursuant to the software. The application 680 and the O/S 650 are read, in whole or in part, by the processor 610, perhaps buffered within the processor 610, and then executed.

When the application 680 is implemented in software it should be noted that the application 680 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 680 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1-3, and the system of Fig. 4, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 4 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions. The architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

**Claims**

1. A method (200) for training a neural network model (250) for predicting an effective energy value of a conventional CT image,
   **characterized in that** the method comprises:
   training a neural network model using a training algorithm (240) configured to receive an array of training inputs (220) and respective known outputs (230), wherein a training input comprises a spectral scanner mono-energetic CT image and a respective known output comprises the effective energy value of the spectral scanner mono-energetic CT image.

2. The method of claim 1, wherein the array of training inputs comprise spectral scanner mono-energetic CT images from 40keV to 140keV with a step-size of 0.1keV or more.

3. The method of claim 1 or 2, wherein the array of training inputs further comprises adjusted spectral scanner mono-energetic CT images, wherein the adjustment comprises slice-flipping, rotating, shifting, zooming, cropping, and using different noise levels.

4. The method of any of claims 1-3, wherein the neural network model comprises a convolutional neural network or a machine learning regression model or a classification model.

5. The method of any of claims 2-4, wherein the array of training inputs further comprise corresponding aggregated feature vectors of each respective spectral scanner mono-energetic CT image.

6. The method of claim 5, wherein the aggregated feature vectors are pre-processed using a principle components analysis, PCA.

7. The method of either of claim 5 or 6, wherein the aggregated feature vectors comprise histograms of the HU values within each respective spectral scanner mono-energetic CT image.

8. A method for estimating an effective energy value of a conventional CT image, the method comprising:

   obtaining (310) a first CT image; and
   processing (330) the first CT image, using the neural network model trained in accordance with any of the methods of any of claims 1 to 7, to generate a first estimated effective energy value of the first CT image.

9. The method of claim 8, wherein the method further comprises:
   using (340) the first estimated effective energy value in a further image-processing step of the first CT image such as a bone beam hardening correction.

10. The method of claim 8 or 9, wherein the method further comprises:

    segmenting (320) the first CT image into multiple CT sub-images; and
    processing the multiple CT sub-images using the neural network model to generate estimated effective energy values for each CT sub-image.

11. The method of any of claims 8-10, further comprising:

    obtaining (410) a second CT image;
    processing (430) the second CT image, using the neural network model, to generate a second estimated effective energy value of the second CT image; and
    calculating (440) a transfer function based on the first estimated effective energy value of the first CT image and the second estimated effective energy value of the second CT image.

12. The method of claim 11, wherein the method further comprises:
    performing (450) a material decomposition based on the first CT image and the second CT image using the calculated transfer function.

13. A computer program comprising code means for implementing the method of any preceding claim when said program is run on a processing system.

14. A system (500) for training a neural network model (250) for predicting an effective energy value of a conventional CT image,
    **characterized in that** the system comprises:
    a processor arrangement (510) configured to:
    train a neural network model using a training algorithm (240) configured to receive an array of training inputs (220) and respective known outputs (230), wherein a training input comprises a spectral scanner mono-energetic CT image and a respective known output comprises the effective energy value of the spectral scanner mono-energetic CT image.

15. A system (520) for estimating an effective energy value of a conventional CT image, **characterized in that** the system comprises:
    a processor arrangement (530) configured to:

obtain a first CT image,
process the first CT image,

use the neural network model trained in accordance with any of the methods of any of claims 1 to 7, to generate a first estimated effective energy value of the first CT image.

**Patentansprüche**

1. Verfahren (200) zum Trainieren eines neuronalen Netzwerkmodells (250) zur Vorhersage eines effektiven Energiewerts eines herkömmlichen CT-Bildes,
**dadurch gekennzeichnet, dass** das Verfahren Folgendes umfasst:
Training eines neuronalen Netzwerkmodells unter Verwendung eines Trainingsalgorithmus (240), der so konfiguriert ist, dass er ein Array von Trainingseingaben (220) und jeweiligen bekannten Ausgaben (230) empfängt, wobei eine Trainingseingabe ein monoenergetisches CT-Bild eines Spektralscanners umfasst und eine jeweilige bekannte Ausgabe den effektiven Energiewert des monoenergetischen CT-Bildes des Spektralscanners umfasst.

2. Verfahren nach Anspruch 1, wobei das Array von Trainingseingaben monoenergetische CT-Bilder eines Spektralscanners von 40 keV bis 140 keV mit einer Schrittweite von 0,1 keV oder mehr umfasst.

3. Verfahren nach Anspruch 1 oder 2, wobei das Array von Trainingseingaben weiter angepasste monoenergetische CT-Bilder eines Spektralscanners umfasst, wobei die Anpassung Spiegeln, Drehen, Verschieben, Zoomen, Beschneiden und Verwenden unterschiedlicher Rauschpegel umfasst.

4. Verfahren nach einem der Ansprüche 1-3, wobei das neuronale Netzwerkmodell ein faltendes neuronales Netzwerk oder ein Regressionsmodell des maschinellen Lernens oder ein Klassifizierungsmodell umfasst.

5. Verfahren nach einem der Ansprüche 2-4, wobei das Array von Trainingseingaben weiter entsprechende aggregierte Merkmalsvektoren jedes jeweiligen monoenergetischen CT-Bildes des Spektralscanners umfasst.

6. Verfahren nach Anspruch 5, wobei die aggregierten Merkmalsvektoren unter Verwendung einer Hauptkomponentenanalyse, PCA, vorverarbeitet werden.

7. Verfahren nach Anspruch 5 oder 6, wobei die aggregierten Merkmalsvektoren Histogramme der HU-Werte innerhalb jedes jeweiligen monoenergetischen CT-Bildes des Spektralscanners umfassen.

8. Verfahren zur Schätzung eines effektiven Energiewerts eines herkömmlichen CT-Bildes, wobei das Verfahren Folgendes umfasst:

Erhalten (310) eines ersten CT-Bilds; und
Verarbeiten (330) des ersten CT-Bildes unter Verwendung des gemäß einem der Verfahren der Ansprüche 1 bis 7 trainierten neuronalen Netzwerkmodells zum Erzeugen eines ersten geschätzten effektiven Energiewerts des ersten CT-Bildes.

9. Verfahren nach Anspruch 8, wobei das Verfahren weiter Folgendes umfasst:
Verwenden (340) des ersten geschätzten effektiven Energiewerts in einem weiteren Bildverarbeitungsschritt des ersten CT-Bildes, wie beispielsweise einer Knochentrabekelhärtungskorrektur.

10. Verfahren nach Anspruch 8 oder 9, wobei das Verfahren weiter Folgendes umfasst:

Segmentieren (320) des ersten CT-Bildes in mehrere CT-Teilbilder; und
Verarbeiten der mehreren CT-Teilbilder unter Verwendung des neuronalen Netzwerkmodells, um für jedes CT-Teilbild geschätzte effektive Energiewerte zu erzeugen.

11. Verfahren nach einem der Ansprüche 8-10, weiter umfassend:

Erhalten (410) eines zweiten CT-Bildes;
Verarbeiten (430) des zweiten CT-Bildes unter Verwendung des neuronalen Netzwerkmodells zum Erzeugen eines zweiten geschätzten effektiven Energiewerts des zweiten CT-Bildes; und

Berechnen (440) einer Transferfunktion basierend auf dem ersten geschätzten effektiven Energiewert des ersten CT-Bildes und dem zweiten geschätzten effektiven Energiewert des zweiten CT-Bildes.

**12.** Verfahren nach Anspruch 11, wobei das Verfahren weiter Folgendes umfasst:

Durchführen (450) einer Materialzerlegung basierend auf dem ersten CT-Bild und dem zweiten CT-Bild unter Verwendung der berechneten Transferfunktion.

**13.** Computerprogramm, das Codemittel zum Implementieren des Verfahrens nach einem vorstehenden Anspruch umfasst, wenn das Programm auf einem Verarbeitungssystem läuft.

**14.** System (500) zum Trainieren eines neuronalen Netzwerkmodells (250) zur Vorhersage eines effektiven Energiewerts eines herkömmlichen CT-Bildes,

**dadurch gekennzeichnet, dass** das System Folgendes umfasst:

eine Prozessoranordnung (510), die konfiguriert ist zum:

Trainieren eines neuronalen Netzwerkmodells unter Verwendung eines Trainingsalgorithmus (240), der so konfiguriert ist, dass er ein Array von Trainingseingaben (220) und jeweiligen bekannten Ausgaben (230) empfängt, wobei eine Trainingseingabe ein monoenergetisches CT-Bild eines Spektralscanners umfasst und eine jeweilige bekannte Ausgabe den effektiven Energiewert des monoenergetischen CT-Bildes des Spektralscanners umfasst.

**15.** System (520) zur Schätzung eines effektiven Energiewerts eines herkömmlichen CT-Bildes, **dadurch gekennzeichnet, dass** das System Folgendes umfasst:

eine Prozessoranordnung (530), die konfiguriert ist zum:

Erhalten eines ersten CT-Bildes,

Verarbeiten des ersten CT-Bildes, Verwenden des gemäß einem der Verfahren nach einem der Ansprüche 1 bis 7 trainierten neuronalen Netzwerkmodells zum Erzeugen eines ersten geschätzten effektiven Energiewerts des ersten CT-Bildes.

## Revendications

**1.** Procédé (200) d'entraînement d'un modèle de réseau neuronal (250) destiné à prédire une valeur d'énergie efficace d'une image de tomodensitométrie conventionnelle,

**caractérisé en ce que** le procédé comprend :

l'entraînement d'un modèle de réseau neuronal à l'aide d'un algorithme d'entraînement (240) configuré pour recevoir un ensemble d'entrées d'entraînement (220) et de sorties connues respectives (230), dans lequel une entrée d'entraînement comprend une image de tomodensitométrie mono-énergétique par scanner spectral et une sortie connue respective comprend la valeur d'énergie efficace de l'image de tomodensitométrie mono-énergétique par scanner spectral.

**2.** Procédé selon la revendication 1, dans lequel l'ensemble d'entrées d'entraînement comprend des images de tomodensitométrie mono-énergétique par scanner spectral de 40 keV à 140 keV avec une taille de pas de 0,1 keV ou plus.

**3.** Procédé selon la revendication 1 ou 2, dans lequel l'ensemble d'entrées d'entraînement comprend en outre des images de tomodensitométrie mono-énergétique par scanner spectral ajustées, dans lequel l'ajustement comprend un retournement de tranches, une rotation, un décalage, un grossissement, un recadrage, et une utilisation de différents niveaux de bruit.

**4.** Procédé selon l'une quelconque des revendications 1-3, dans lequel le modèle de réseau neuronal comprend un réseau neuronal convolutif ou un modèle de régression par apprentissage automatique ou un modèle de classification.

**5.** Procédé selon l'une quelconque des revendications 2-4, dans lequel l'ensemble d'entrées d'entraînement comprend en outre des vecteurs de caractéristiques agrégés correspondants de chaque image respective de tomodensitométrie mono-énergétique par scanner spectral.

**6.** Procédé selon la revendication 5, dans lequel les vecteurs de caractéristiques agrégés sont prétraités au moyen d'une analyse en composantes principales, PCA.

**7.** Procédé selon la revendication 5 ou 6, dans lequel les vecteurs de caractéristiques agrégés comprennent des histogrammes des valeurs HU dans chaque image respective de tomodensitométrie mono-énergétique par scanner spectral.

**8.** Procédé d'estimation d'une valeur d'énergie efficace d'une image de tomodensitométrie conventionnelle, le procédé comprenant :

l'obtention (310) d'une première image de tomodensitométrie ; et
le traitement (330) de la première image de tomodensitométrie, au moyen du modèle de réseau neuronal entraîné d'après l'un quelconque des procédés selon l'une quelconque des revendications 1 à 7, de façon à générer une première valeur d'énergie efficace estimée de la première image de tomodensitométrie.

**9.** Procédé selon la revendication 8, dans lequel le procédé comprend en outre :
l'utilisation (340) de la première valeur d'énergie efficace estimée au cours d'une étape de traitement d'image supplémentaire de la première image de tomodensitométrie telle qu'une correction d'un durcissement du faisceau au niveau des os.

**10.** Procédé selon la revendication 8 ou 9, dans lequel le procédé comprend en outre :

la segmentation (320) de la première image de tomodensitométrie en multiples sous-images de tomodensitométrie ; et
le traitement des multiples sous-images de tomodensitométrie au moyen du modèle de réseau neuronal de façon à générer des valeurs d'énergie efficace estimées relatives à chaque sous-image de tomodensitométrie.

**11.** Procédé selon l'une quelconque des revendications 8-10, comprenant en outre :

l'obtention (410) d'une seconde image de tomodensitométrie ;
le traitement (430) de la seconde image de tomodensitométrie, au moyen du modèle de réseau neuronal, de façon à générer une seconde valeur d'énergie efficace estimée de la seconde image de tomodensitométrie ; et
le calcul (440) d'une fonction de transfert sur la base de la première valeur d'énergie efficace estimée de la première image de tomodensitométrie et de la seconde valeur d'énergie efficace estimée de la seconde image de tomodensitométrie.

**12.** Procédé selon la revendication 11, dans lequel le procédé comprend en outre :
l'exécution (450) d'une décomposition des matières sur la base de la première image de tomodensitométrie et de la seconde image de tomodensitométrie au moyen de la fonction de transfert calculée.

**13.** Programme informatique comprenant des moyens de code permettant de mettre en œuvre le procédé selon une quelconque revendication précédente lorsque ledit programme est exécuté sur un système de traitement.

**14.** Système (500) d'entraînement d'un modèle de réseau neuronal (250) destiné à prédire une valeur d'énergie efficace d'une image de tomodensitométrie conventionnelle,
**caractérisé en ce que** le système comprend :
un agencement de processeur (510) configuré pour :
entraîner un modèle de réseau neuronal au moyen d'un algorithme d'entraînement (240) configuré pour recevoir un ensemble d'entrées d'entraînement (220) et de sorties connues respectives (230), dans lequel une entrée d'entraînement comprend une image de tomodensitométrie mono-énergétique par scanner spectral et une sortie connue respective comprend la valeur d'énergie efficace de l'image de tomodensitométrie mono-énergétique par scanner spectral.

**15.** Système (520) d'estimation d'une valeur d'énergie efficace d'une image de tomodensitométrie conventionnelle,
**caractérisé en ce que** le système comprend :
un agencement de processeur (530) configuré pour :

obtenir une première image de tomodensitométrie,

traiter la première image de tomodensitométrie, au moyen du modèle de réseau neuronal entraîné d'après l'un quelconque des procédés selon l'une quelconque des revendications 1 à 7, de façon à générer une première valeur d'énergie efficace estimée de la première image de tomodensitométrie.

FIG. 1

200

210 — Training a neural network model using a training algorithm

FIG. 2A

220

230

Training inputs

Respective Known Outputs

240 — Training Algorithm

250

FIG. 2B

300

310 — Obtaining a first CT image

320 — Segmenting first CT image into multiple CT sub-images

330 — Estimating effective energy value of first CT image (or sub-images) using neural network model

340 — Further processing of the first CT image using estimated effective energy value

**FIG. 3**

400

310
Obtaining a first CT image

410
Obtaining a second CT image

420
Obtaining a third CT image

430 — Estimating effective energy values of the CT images using neural network model

440 — Calculating transfer function based on the estimated effective energy values

450 — Performing material decomposition based on the CT images

**FIG. 4**

500

510

Processor Arrangement

520

530

Processor Arrangement

# FIG. 5

610   660   670   600

μP

650   O/S

I/O

App

630   680   620

**FIG. 6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7968853 B2

### Non-patent literature cited in the description

- **MILLNER et al.** Determination of effective energies in CT calibration. *Med Phys.*, November 1978, vol. 5 (6), 543-5
- **LI YINSHENG et al.** A quality-checked and physics-constrained deep learning method to estimate material basis images from single-kV contrast-enhanced chest CT scans. *Medical Physics.*, 23 March 2023, vol. 50 (6), ISSN 0094-2405, 3368-3388
- Experimental investigation of neural network estimator and transfer learning techniques for K-edge spectral CT imaging. *Medical Physics*, 06 January 2020, vol. 47 (2), ISSN 0094-2405, 541-551